# EUROPEAN PATENT APPLICATION

(11) **EP 1 779 812 A1**
(43) Date of publication of application: **02.05.2007**
(21) Application number: 05110030.3
(22) Date of filing: 26.10.2005
(51) Int. Cl.: A61F 2/30

(54) **An osseointegration implant**

(71) Applicant: Etervind AB, 192 69 Sollentuna (SE)
(72) Inventor: Axelsson, Robert, 563 91, Gränna (SE); Trepte, Oliver, 19269 Sollentuna (SE)
(74) Representative: Holme Patent A/S

(57) **Abstract**

The invention relates to an osseointegration implant (1) comprising a three-dimensional structure, which at least partly covers the surface of the implant. The three-dimensional structure is a latticework (4), and the ratio of the volume of interstices (10,11,14,15) of the latticework to the volume of the mass of the latticework is increasing towards the bone contacting face (6). Such a latticework (4) matches the characteristics of natural bone and assures that the requisite amount of loading is transferred to the surrounding bone. This will further minimize the stress applied to the interface between the host tissue and the implant eliminating atrophy resulting from either insufficient or excessive loading. This will result in both reduced surgical cost and complications associated with implantation.

## Description

The present invention relates to an osseointegration implant comprising an open three-dimensional structure at least partly covering its surface. The invention also relates to the use of the implant and to a method for manufacturing the implant according to the invention.

It is generally known in the art to use prosthetic implants to replace portions of the human anatomy that have been damaged due to injury or age.

Fractures is for example commonly be repaired with a bone plate which spans the break and damaged joints can be replaced with a functional joint prosthesis to provide motion and to reduce deterioration of the adjacent bone and adjacent joints.

In each case, the structural element of the prosthetic repair takes over a portion of the natural mechanical loading of the bone, but also requires regrowth of that bone in order for the prosthetic implant to become effectively attached to or be incorporated in the bone.

However, most of these implants only last up to about 10 to 15 years, as many implants become "loose" within the bone, which eventually leads to implant failure.

Implant failure is be due to many factors, including surgery techniques, patient activity and weight, wear of the implant and osteolysis, but one of the largest problems observed relates to improper distribution of stresses within the implant and throughout the surrounding bone.

It is well known in the art, that healthy bone undergoes growth processes, which compensates normal bone resorption or wasting. Bone growth or accretion further operates to maintain the requisite bone strength. Moreover, bone growth increases when the bone is subject to mechanical stress.

Conversely, when a prosthetic implant or bone plate takes over a portion of the loading on a bone, as the elastic modulus of a prosthetic stem or bone plate typically is much higher than that of the bone it replaces, portions of bone may experience no natural loading, and the bone can be resorbed or broken down by the body, resulting in atrophy, i.e. bone loss for the patient. The phenomenon of atrophy resulting from insufficient loading at certain portions of the implant and bone interface has been referred to as stress shielding.

On the other hand, if the bone is required to take on too much stress, abnormal growth can occur- called "adaptive remodelling." Too much stress may also lead to resorption or may result in an undesirable atrophy of the affected area. Furthermore, excessive stress loadings can ultimately result in bone fractures.

Stress shielding has previously been addressed by putting a homogenous porous coating or three-dimensional structure on at least part of the implant. The porous coating is utilized to encourage the growth of hard tissue around the implant.

Implants of this type have been in clinical use for a number of years, e.g. as an aid in hip arthroplasty procedures in order to alleviate the symptoms of trauma or degeneration of the hip. In contrast to the cementable implants, the fixation of these implants occurs without cement, since the bone grow into the porous coating and ossify there. Implants of this type are known, for example, from DE-A1-29 10 627, DE-A1-31 06 917 and from DE-U1-90 11 363.

However, these implants have not been successful in the attempt to duplicate the natural transmission of the load in e.g. the long bones, and are associated with an undesirable atrophy of the affected areas.

Furthermore, the inventors has found that e.g. the trabecular bones, as an integral part of the natural spongiosa, cannot grow well into the homogenous structure of the uniform surface structure of these known implants. Ultimately, this leads to a prolongation of the healing phase, the reconstruction phase of the bony layers into the implant and to a shortening of the long-term fixation.

Other attempts to reduce stress shielding include various attempts to make the stem more flexible. However, these efforts are expensive and in each case allow the distal portion of the prosthesis to rub against the inside of the bone canal as the bone flexes. This may be painful to the patient and may induce undesired bone growth near the distal end of the prosthesis as the prosthesis rubs against the inside of the bone canal.

Accordingly, there exists a long-term need for an improved osseointegration implant, which addresses the needs and problems of stress distribution in the implant and to the surrounding bone.

In view of this background, it is a first aspect of the present invention to create an implant having the characteristics of natural bone and minimizing the stress applied to the interface between the host tissue and the implant.

It is a second aspect of the present invention to create an implant, which assures that the requisite amount of loading continues to be transferred to surrounding bone to encourage the receiving residual bone to grow strong and bind to the implant.

It is a third aspect of the present invention to provide an implant with an three-dimensional structure at least partly covering its surface, whose healing ability, as well as whose long-term fixation properties, are a clear improvement over the known implants.

It is a forth aspect of the present invention to provide an implant in which the density of the three-dimensional structure substantially matches the mechanical properties of bone, such as strength and modulus of elasticity.

It is a fifth aspect of the present invention to provide an implant having interconnecting pores and channels to facilitate invasion of cells into the three dimensional structure and/or a nutrient flow to cells.

The novel and unique whereby this is achieved according to the present inventions is the fact that the three-dimensional structure is a latticework, and that the ratio of the volume of interstices in the latticework to the volume of the mass of the latticework is increasing towards the bone contacting face.

By placing a latticework on an implant with a perpendicular gradient of the ratio of the volume of interstices of the latticework to the volume of its mass, i.e. that different interstices volumes are represented in the spatial depth of the latticework, the latticework matches the characteristics of natural bone and assures that the requisite amount of loading continues to be transferred to the surrounding bone whereby the natural transmission of the load in e.g. the long bones is duplicated.

This will further minimize the stress applied to the interface between the host tissue and the implant and the phenomenon of atrophy resulting from either insufficient or excessive loading at certain portions of the implant and bone interface is eliminated, resulting in both reduced surgical cost and complications associated with implantation of the implant.

Furthermore, due to the structure of a latticework the implant according to the invention will furthermore have a more even distribution of forces, and improved properties over the prior art with respect to stress properties, elastic adaptation, and structural compatibility in order to achieve faster bone tissue ingrowth.

The implant according to the invention preferably rely on tissue integration instead of e.g. screws or cement for stability, as this will ensure a better long-term stability af implantation of the implant. However, the resulting implant will be able to function in both press-fit and cemented situations, and in some cases, a combination of fixation techniques is used.

It will be understood by the person skilled in the art that by latticework is meant a lattice or lattice-like structure, such as an open, crisscross pattern or weave

The interstices of the latticework are adventurously interconnected, meaning that all interstices of the latticework are connected either directly or indirectly, and that there are no closed cavities.

The latticework will thus be a three dimensional network of continuously connected interstices and channels which define a void volume. This void volume is preferably approximately 50-95%, and preferably at least 80%. Such a latticework provides optimal permeability and a high surface area, which gives ideal conditions for the growth of new bone, as this structure provides prompt and easy access for e.g. cells, blood vessels and nutrients. Additionally, the latticework implants promote a stable bone implant interface by the immigration of bone-forming cells from the surrounding bone.

Furthermore, transmitted loads are distributed over a larger area than with less open structures, thereby minimizing the stress applied to the interface between the host tissue and the implant.

According to an advantageous embodiment of the implant, the latticework is a multilayer structure, in which the volume of the interstices is increasing in each layer, starting from the layer closest to the surface of the implant, providing both structural strength and resiliency together with a stable bone implant interface in a single implant.

This means, that different interstices volumes are represented in the spatial depth of the latticework. The inventors have surprisingly found, that such a structure is especially good at minimizing the stress applied to the interface between the host tissue and the implant.

By encouraging bone ingrowth into every interstices of the latticework a structure in which forces are transmitted from every portion of the bone to every portion of the implant is achieved. Thus, the load is distributed evenly in the bone and implant, eliminating the tendency for any particular part of the bone to become subjected to atrophy and the consequent loosening of the implant, and at the same time ensuring, that the bone is not subjected to excessive load.

The volumes of the interstices are preferably adapted to the cell and pore structure of the bone material, which is in contact with the surface structure of the implant after the implantation. This means that the respective volume of the interstices correspond approximately in size to the cells and pores of the natural spongiosa, which should grow into and onto the surface structure of the implant. For the spongiosa with a small cell and pore size, there will accordingly be provided a layer having a correspondingly smaller volume of the interstices closest to the surface of the implant. Similarly, for a spongiosa with a large cell and pore size, there will be provided a layer on the implant with a correspondingly larger volume of the interstices.

The cell and pore sizes of the natural spongiosa in bones of e.g. humans can be catalogued, so that the implants can be produced with corresponding zones of different volume of the interstices, depending on which bone part the implant is intended for.

The construction of the implant according to the invention has the advantage that a given cell can grow into the latticework and meet with a corresponding interstices having similar size ensuring a significantly improved growth relationship of the implant. Furthermore, the implant of the invention improves the properties relating to a long-term fixation; since the interactions of newly formed hard tissue in or around the latticework of the implant has been found to provide a good fixation for the prosthesis within the bone.

The inventors has surprisingly found, that if the latticework has a thickness of about 0,5 to about 2,5 mm, depending on the use of the implant, the desired stress properties, elastic adaptation and structural compatibility in order to achieve faster bone tissue ingrowth, is achieved.

In a preferred embodiment, the size of interstices are desirably at least 80 µm and preferably in the range of 100 µm to 500 µm. The smallest interstices sizes are provided closest to the surface of the implant, i.e. farthest from the interface with the bone. The sizes of the interstices promote easy and prompt access for cells, blood vessels, and nutrients thereby enhancing new bone formation.

The latticework preferably has a predetermined configuration to the structure, and in a first embodiment the latticework is a scaffold, i.e. a strong supportive framework of struts providing a plurality of interconnecting interstices, the size of which increase throughout the bulk volume of the scaffold towards the interface with the host tissue.

In a second embodiment according to the invention the latticework is in the form of superposed corrugated sheets. This embodiment has in computer simulations proven to be especially good at minimizing the stress applied to the interface between the host tissue and the implant and consequently for distributing stress and forces evenly throughout the entire implant. This embodiment is therefore capable of duplicating the natural transmission of the load in e.g. the long bones, reducing the risk of atrophy in the affected areas. The result is an implant with the favourable qualities a Young's module close to that of bone, and which at the same time has a high elastic limit and breakage point.

It is believed that the corrugated structure of the latticework enhances the strength of the implant. More particularly, the corrugated latticework will exhibit load bearing properties superior to those of non-corrugated latticework, as the implant will exhibit greater resistance to compression, bucking and bending. Practically this means that the layer thickness of a corrugated latticework can be thinner than the thickness of a noncorrugated latticework, while exhibiting substantially the same strength properties.

Structurally, the latticework may be solid and dense, or may be porous. Solid, dense latticework provides a higher modulus of elasticity and exhibit greater strength than a porous latticework. However, the lower modulus of elasticity of porous latticework more closely approaches the modulus of elasticity of natural bone.

As stress shielding occurs when a difference in modules between materials changes the stress distribution in the bone, generally reducing the load in the bone, it is desirable to have a material with a modulus of elasticity which is closer to that of cortical bone, and which at the same time has a high elastic limit and breakage point and good biocompatibility resulting in a loss of bone.

Modulus of elasticity or Young's modulus is a measure of the stiffness of a given material. It is defined as the limit for small strains of the rate of change of stress with strain. This can be experimentally determined from the slope of a stress-strain curve created during tensile tests conducted on a sample of the material.

Due to the void volume of the latticework according to the present invention, a Young's module close to that of e.g. cortical bone, and which at the same time has a high elastic limit and breakage point can be provided in a single implant.

The geometry of the interstices of the latticework can preferably be polyhedron-like, and although different geometries of the interstices can be mixed together and employed in the invention, it is desired that the interstices in the latticework be of a substantially uniform geometry to provide proper distribution of forces and ingrowths of cells.

Examples of preferably polyhedrons, which can be used in the present invention, are: prismatoid, cubic, tratragonals, rhombohedral, orthorhombic, but other geomical shapes are also anticipated within the scope of invention. The diversity of the possible shapes of the interstices ensures, that the latticework can be shaped into customized implants, fitting e.g. small implant site such as a finger or mandible, or to be contoured to a desired topography of an anatomical site, and still take different cell-types of the host tissue into consideration.

The implant and latticework can preferably be made of the same material, and preferably of a biologically material compatible with human bone tissue. As examples of useable materials can be mentioned titanium alloys, elemental titanium, and chromium-cobalt alloys.

Titanium and its alloys exhibit excellent mechanical and biological properties: For cellular materials, stiffness drops with the square of relative density (Gibson, L., and Ashby, M., 1997. Cellular solids: Structure and properties.), meaning, that a latticework of titanium is expected to show a much reduced stress shielding effect as compared to current monolithic metallic implants exhibiting stiffness values up to ten times higher than that of the hosting bone. Additionally, the latticework implants promote a stable bone implant interface by the immigration of bone-forming cells from the surrounding bone.

The latticework according to the invention may be coated at least partially with a preparation inducing formation of new bone, such as an osteobiologic agent. The person skilled in the art is familiar with such preparations, however, as one example can be mentioned, that the bone morphogenic protein, BMP-7, sold as OP-1 by Stryker Biotech, can be appropriate for this purpose.

The invention also relates to a method for producing an implant according to the invention having a latticework comprises at least two layers, said method comprising the successive steps of:
- applying to at least a portion of the surface of said implant a first layer comprising openings having a first size, and
- applying to said first layer a second layer having openings with a second size, and wherein said second size is lager than said first size.

If more than two layers are desired, each new layer, which preferably will have openings which are larger than the openings in the previously layer, are applied in a similar manner as the first two layers.

The layers are applied to the surface of the implant by means of conventional technique such that the layers after application appear as the layers are in one piece with the remaining implant. Commonly, the layers is sintered, diffusion bonded, or welded to the implant. These processes require heating the implant and layers to a temperature sufficient to cause the different layers and the implant body to fuse, melt or bond together at their point of mutual contact. One method, which can be used for applying the layers to the implant, is disclosed in US 6,576,014.

If only a part of the implant is to be covered with the latticework, it is desirable to provide a recess in the implant for the latticework, ensuring that the bone contacting face of the latticework and the remaining surface of the implant are in the same plane.

The implant according to the invention can be used as a prosthetic implants to replace portions of the human anatomy that have been damaged due to injury or age, e.g. a bone joint, or for splicing or repairing a traumatic bone injury.

The invention will now be described by way of example only with reference to the drawing, in which
Fig. 1 shows a perspective view of a first embodiment of the implant according to the present invention,
Fig. 2 shows a cross-section of the embodiment shown in figure 1,
Fig. 3 shows a perspective view of a second embodiment of the implant according to the present invention, and
Fig. 4 shows a cross-section of the embodiment shown in figure 3.

The invention is described below by way of example with the assumption that the implant is replacing a joint, such as the hip, however within the scope of the invention the implant can be used to replace other portions of the human anatomy that have been damaged due to injury or age, or for splicing or repairmen of traumatic bone injury.

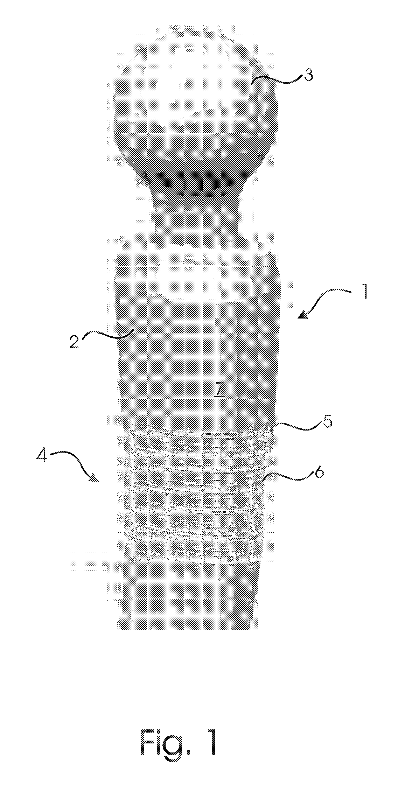

Figure 1 shows part of a conventional femoral implant 1. The femoral implant generally consists of a radial femoral stem section 2 and a rounded femoral cap 3. The femoral stem section is provided with, as schematically displayed, a latticework 3 which partly covers the surface of the implant 1.

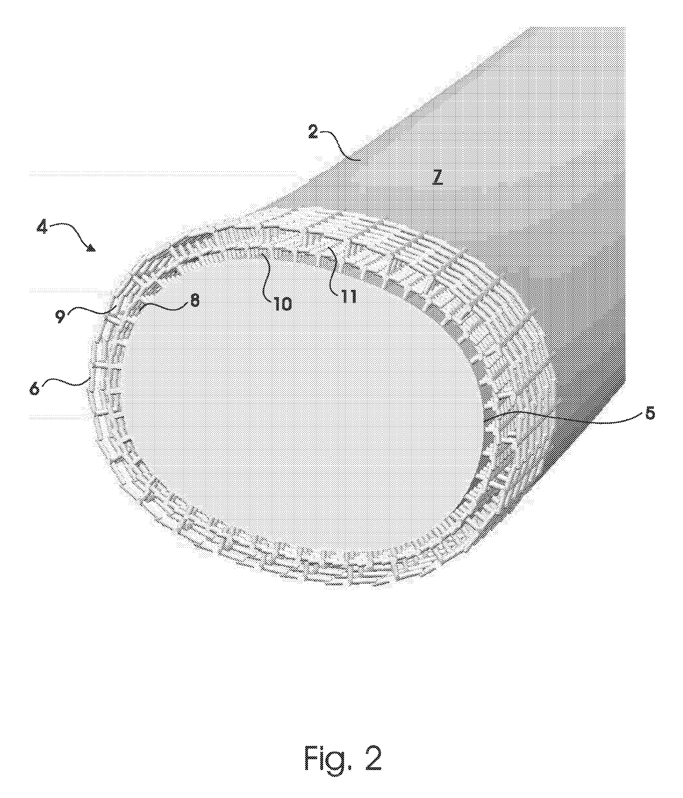

In figure 1 and 2 the latticework 4, is in the form of a scaffold, and have been placed in a recess 5 in the femoral stem section 2. This ensures, that the bone contacting face of the latticework 6 and the surface of the femoral stem section 7 are in the same plane.

As best seen in figure 2, the scaffold 4 is divided into two discrete layers 8 and 9, both layers having a plurality of interstices 10 and 11, respectively. The interstices all have the substantially same shape of prismatoids, providing both a simple and efficient way of ensuring proper distribution of forces and ingrowths of cells.

The ratio of the volume of interstices 10 in the layer 8 closest to the surface of the femoral stem section 2, to the volume of the mass of layer 8 is smaller, than the volume of interstices 11 in layer 9, to the volume of the mass of layer 9.

The scaffold 4 structure shown in figure 1 and 2 provides a strong supportive framework of struts defining a plurality of interconnecting interstices 10, 11, the volume of which increase throughout the bulk volume of the scaffold 4, starting with the smallest volume near the surface of the recess 5 ending in the largest volume near the bone contacting face 6 of the scaffold 4.

Thus, the largest void volume is placed in the outer layer, 9, and the smallest void volume in the layer closest to the implant, ensuring that different volumes of interstices are represented in the spatial depth of the scaffold, whereby the stress applied to the interface between the host tissue and the implant is minimised.

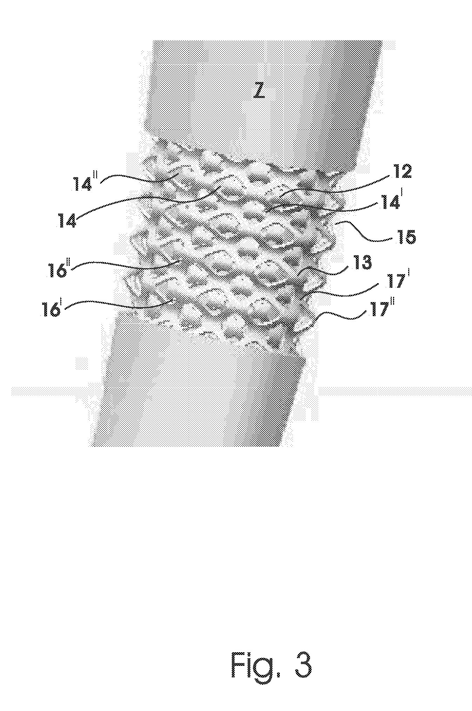

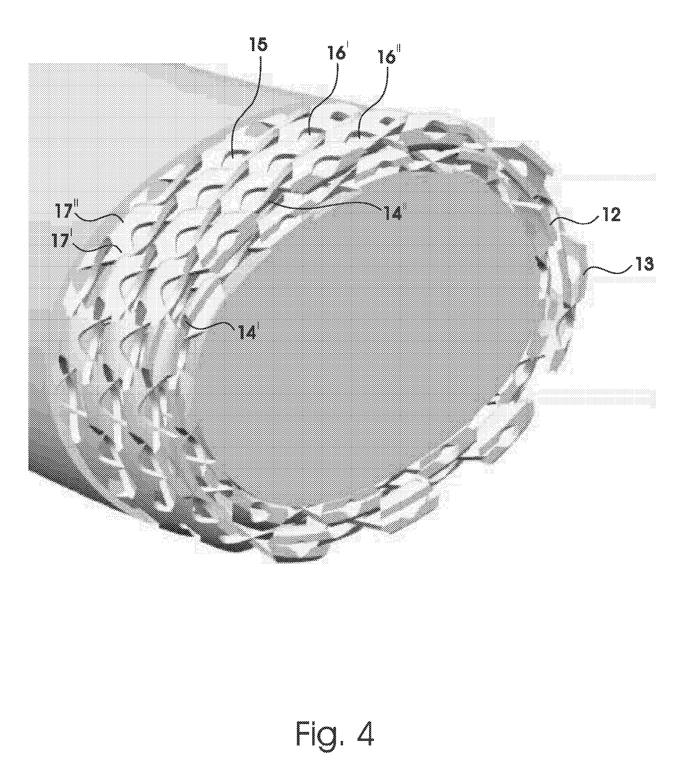

Figure 3 and 4 shows a second embodiment of the latticework 4 according to the present invention. In this embodiment the latticework is in the form of two superposed corrugated sheets 12, 13. As is evident from the drawings, each layer 12, 13 have a corrugated structure, in which a plurality of interstices, 14, 15 respectively, is provided in a harlequin-like pattern throughout the corrugated structure. Each layer has a plurality of notes 16', 17' placed in the vales of the layers 12, 13, and a plurality of notes 16", 17" places in the crests of the layers 12, 13.

Correspondingly the interstices 14, 15 of the layers will be places in the vales, 14', 15' respectively, and in the crests 14", 15", respectively.

The two layers 12, 13 are lateral displaced in respect to each other providing a structure in which e.g. the notes 16" places in the crests of the inner layers 12 flushes with the interstices 14', 14" places at both vales and crests of the outer layer 13.

As in the first embodiment the ratio of the volume of interstices 14 in the layer 12 closest to the surface of the femoral stem section 2, to the volume of the mass said layer 12 is smaller, than the respective ratio in the outer layer 13.

This provides a latticework 4 where the void volume increases throughout the bulk volume of the latticework. This structure ensures, that all interstices 14, 15 of the latticework are connected either directly or indirectly, and that there are no closed cavities. This will facilitate invasion of cells into the three dimensional structure and/or a nutrient flow to cells.

Furthermore, the corrugated structure of the latticework enhances the strength of the implant, ensuring that the implant will exhibit greater resistance to compression, bucking and bending. Thus, thinner layers 12, 13 can be used while exhibiting substantially the same strength properties as non-corrugated layers. This provides the possibility of using smaller implants than hitherto known.

The latticework shown in figure 3 and 4 is especially good at minimizing the stress applied to the interface between the host tissue and the implant and consequently for distributing stress and forces evenly throughout the entire implant. This embodiment is therefore capable of duplicating the natural transmission of the load in e.g. the long bones, reducing the risk of atrophy in the affected areas.

The use of the latticework 4 having a graded void volume as shown in the figures, is advantageously because such a structure resembles the microstructure of natural cancellous bone and therefore will enhance incorporation of new bone into the latticework.

As bone material grows into the latticework after the implantation, this will not only causes permanent fixation of the implant to the bone but also ensure that forces are transmitted from every portion of the bone to every portion of the implant. Thus, the load is distributed evenly in the bone and implant, eliminating the tendency for any particular part of the bone to become subjected to atrophy and the consequent loosening of the implant.

The selection of interstices volume of the layers 8, 9 and 12, 13 in the latticework 4 depend on the natural cell and pore widths (diameters) of the spongiosa in the femur bone.

A femoral implant 1 bears several kinds of load. At times, the load can exceed three times the weight of the body. Although some flexural and tensional stresses may be applied to the implant, the principal loads placed thereon in the human body are likely to be compressive and shear stresses. In a joint such as the hip, the shear stresses are the most important.

The implant according to the present invention is particularly well adapted to handle shear stresses without failing at the bone contacting face of the latticework. The graded interstices in the latticework encourage bone ingrowths, thereby producing a physical locking effect. Not only does the bone interlock with the latticework, but it also fills the voids and strengthens the latticework.

When the bone substantially fills the voids in the latticework, the resultant structure becomes exceedingly strong, while at the same time ensuring, that the implant is matching the characteristics of natural bone. Thereby will the requisite amount of loading continue to be transferred to the surrounding bone, duplicating the natural transmission of the load in the long bone and ensure that the phenomenon of atrophy resulting from either insufficient or excessive loading at certain portions of the implant and bone interface is eliminated.

### EXAMPLE

A comparison between two implant designs regarding structural mechanics has been performed.

The implant design analysed are a traditional design as disclosed in DE 10202871 to Eska Implants GmbH & Co, and a latticework according to the invention in the form of superposed corrugated sheets, as disclosed in figure 3 and 4.

FEM models were used to calculate the strength of the two implants. Sections of the implants (approx 5x7 mm) have been modelled with a high stiffness (Young's modulus 200 GPa) and the "bone" with a low stiffness (70 GPa). Contacts between the "bone" and implants have been assumed and a unit pulling pressure on the bones (4 MPa) has been applied and the stresses in the implants have been analysed. The analyses are for comparative purposes only and only consider structural mechanics.

From the calculations it is obvious that the design according to the invention distributes the stresses more evenly over the implant geometry; the top locations of the implant geometry are exposed to stresses almost as much as the bottom regions closer to the body. This explains the fact that the stress concentrations in this design are less pronounced. The opposite observation can be made on the Traditional Implant Design; the top regions of the implant are exposed to low stresses hence increasing the stresses in the regions closer to the body.

The implant according to the invention gives a more favourable stress distribution on the bone, strengthening the overall joint between the implant and bone, which in turn reduces the risk of bone rupture.

## Claims

1. An osseointegration implant (1) comprising a three-dimensional structure which at least partly covers the surface of the implant, **characterized in, that** the three-dimensional structure is a latticework (4), and that the ratio of the volume of interstices (10,11,14,15) of the latticework to the volume of the mass of the latticework is increasing towards the bone contacting face (6).

2. The implant (1) according to claim 1, **characterised in, that** the interstices (10,11,14,15) of the latticework (4) are interconnected.

3. The implant (1) according to claim 1 or 2, **characterised in, that** the latticework (4) is a multilayer structure.

4. The implant (1) according to claim 3, **characterised in, that** the volume of interstices (10,11,14,15) in the layer closest to the surface (8, 12) of the implant are not less than about 80 µm.

5. The implant (1) according to claims 1, 2 or 3, **characterised in, that** the latticework (4) is the form of a scaffold.

6. The implant (1) according to claims 1, 2 or 3, **characterised in, that** the latticework (4) is in the form of superposed corrugated sheets (12,13).

7. The implant (1) according to claims 1 - 6, **characterised in, that** the geometry of the interstices (10,11) of the latticework (4) are substantially polyhedron.

8. The implant (1) according to claim 1 - 7, **characterised in, that** the latticework (4) are of the same material as the implant, and said structure and implant both are biologically compatible with human bone tissue.

9. The implant (1) according to claim 8, **characterised in, that** the material is selected from the group comprising stainless steel, titanium alloys, elemental titanium, and chromium-cobalt alloys.

10. The implant (1) according to claim 1-9, **characterised in, that** the latticework (4) has a thickness of about 0,5 to about 1,5 mm.

11. A method of manufacturing the osseointegration implant (1) according to any of the claims 3-10, wherein the latticework (4) comprises at least two layers, **characterized in, that** the method comprising the successive steps of:
- applying to at least a portion of the surface (5) of said implant a first layer (8,12) comprising interstices (10, 14) having a first size, and
- applying to said first layer (8,12) a second layer (9,13) having interstices (9,15) with a second size, said second size is lager than the first size.

12. Use of the implant (1) according to claim 1 - 9 for replacement of a bone joint, or the splicing or repair of traumatic bone injury.
